(19) **Europäisches Patentamt**

**European Patent Office**

**Office européen des brevets**

(11) **EP 1 251 202 B1**

(12) **EUROPEAN PATENT SPECIFICATION**

(45) Date of publication and mention
of the grant of the patent:
**14.06.2006  Bulletin 2006/24**

(51) Int Cl.:
*D06M 13/224* (2006.01)    *D06M 13/165* (2006.01)

(21) Application number: **02252732.9**

(22) Date of filing: **17.04.2002**

(54) **Method for manufacturing a functionalized fiber**

Verfahren zur Herstellung eines funktionalisierten Fasermaterial

Méthode pour la production d'un matériau fibreux fonctionalisé

(84) Designated Contracting States:
**DE ES FI FR GB SE**

(30) Priority: **18.04.2001  JP 2001119072**

(43) Date of publication of application:
**23.10.2002  Bulletin 2002/43**

(73) Proprietor: **Fuji Spinning Co., Ltd.
Tokyo (JP)**

(72) Inventors:
• **Itoyama, Koki
  Sunto-gun,
  Shizuoka-ken (JP)**
• **Fujii, Takatoshi,
  No.5 Minamiyama Flat
  Kozakai-cho,Houi-gun,
  Aichi-ken 441-0105 (JP)**
• **Tanibe, Hiroaki
  Gotenba-shi,
  Shizuoka-ken (JP)**

(74) Representative: **W.P. Thompson & Co.
Eastcheap House
Central Approach
Letchworth Garden City,
Hertfordshire SG6 3DS (GB)**

(56) References cited:
**WO-A-00/04230**

• **DATABASE WPI Section Ch, Week 199543
Derwent Publications Ltd., London, GB; Class
A96, AN 1995-332480 XP002208237 & JP 07
228537 A (SAITAMA DAIICHI SEIYAKU KK), 29
August 1995 (1995-08-29)**
• **DATABASE WPI Section Ch, Week 198908
Derwent Publications Ltd., London, GB; Class
A84, AN 1989-057296 XP002208238 & JP 01
008996 A (HITACHI LTD), 12 January 1989
(1989-01-12)**
• **DATABASE WPI Section Ch, Week 199314
Derwent Publications Ltd., London, GB; Class
A96, AN 1993-111902 XP002208239 & JP 05
049551 A (JEX KK), 2 March 1993 (1993-03-02)**

**Description**

Technical Field

The present invention relates to functionalized fiber materials and provides a method of manufacturing a fiber material having a function of sustained discharge of a skin care component with resistance to washing, as well as being superior in feel and moisture absorption and release properties. The fiber material can be utilized in the clothing field such as in underwear and shirts.

Description of the Related Art

α-tocopherol is a natural antioxidant agent, and widely used in cosmetics and health foods for the purposes of skin care and maintaining health. Attempts have already been made to apply α-tocopherol to fiber products for skin care purposes or to provide an antioxidant function. However, since α-tocopherol is oxidized and loses its antioxidant function in a short time if a fiber is simply treated with it, it has become important to stabilize α-tocopherol.

For example, JP-A-10-331070 discloses an antioxidant fiber material, wherein an antioxidant agent is stabilized by forming a complex with a protein followed by treating fiber products with this complex. However, although the antioxidant fiber material is superior for use in the field of food packaging materials, it is in need of improvement in feeling for application to the clothing field. The disclosure has no description of application to the clothing field. In addition, although the antioxidant agent is stabilized and has resistance to washing, it does not have the property of being sustainedly discharged by the action of a fat and oil component such as sebum, even if the antioxidant fiber material were used in the clothing field.

JP-A-11-172522 also discloses a method for adding an antioxidant agent to a regenerated cellulose fiber, wherein the antioxidant agent is stabilized by forming a complex between the antioxidant agent and a protein, then the complex is added to and mixed in a cellulose viscose solution to be spun. In this method in which a complex is formed between an antioxidant agent and a stabilizer such as a protein before being blended in a cellulose viscose solution to be used for manufacturing a fiber, the antioxidant agent is stabilized and has a superior resistance to washing but hardly expresses an effect in wearing when applied to clothing because it takes a long time for the complex to migrate to the fiber surface due to its being incorporated inside the fiber. Also, the antioxidant agent does not have the property of being sustainedly discharged by the action of a fat and oil component such as sebum.

JP-A-10-131042 discloses a fiber treatment agent in which an antioxidant agent such as tocopherol is dispersed in a water phase. The disclosure describes that this fiber treatment agent added to clothing as a finishing agent can suppress a smell of sweat in wearing soon after washing. Also, the deodorizing effect lasts long. The disclosure does not however give any consideration to deterioration of the antioxidant agent when the clothing is left for a long time after washing.

Further, JP-A-2000-110067 discloses that a fiber to which α-type tocopherol in a ratio of 0.01 to 0.5 parts by weight of α-type tocopherol per 100 parts by weight of fiber is adhered has the effect to quicken circulation of the blood and suppress rashes. The fiber is however mainly used for throwaway fiber products because it has no resistance to washing.

WO00/04230 discloses the use of PIT emulsions, containing (a) $C_8$-$C_{22}$ fatty acid alkyl esters; (b) $C_8$-$C_{22}$ fatty alcohols; (c) $C_8$-$C_{22}$ alcohol polyglycol ether and (d) $C_8$-$C_{22}$ fatty acid partial glyceride as impregnating and softening agents for papers, non-wovens fabrics and tissues.

According to the present invention, there is provided a method for manufacturing a functionalized fiber material comprising treating a fiber material with a liquid in which α-tocopherol acetate is emulsified in water with a surfactant, wherein the surfactant is an anionic surfactant alone or a mixture of an anionic surfactant and a nonionic surfactant, and the concentration of the α-tocopherol acetate in the emulsified liquid is 0.5 to 100.0 g/l, to obtain the functionalized fiber material to which 0.3 to 45.0 mg/g of α-tocopherol acetate is adhered for exhibiting a skin-care effect.

Preferably, the α-tocopherol acetate is emulsified in water with the surfactant and an organic solvent.

Conveniently, the organic solvent comprises one or more of oleic acid, squalane and derivatives thereof, hexane, dimethylether, ethyl acetate and dodecanol.

There is disclosed herein a functionalized fiber material in which α-tocopherol acetate is adhered to a fiber material, wherein α-tocopherol acetate is preferably present in an amount of 0.3 mg to 45.0 mg per 1 g of fiber material. There is also disclosed herein a method for treating a fiber material with an emulsion of α-tocopherol acetate having a concentration of 0.5 to 100.0 g/l of α-tocopherol acetate which is emulsified using a surfactant, and a manufacturing method wherein the surfactant used for the emulsification is an anionic surfactant alone or a mixture of an anionic surfactant and a nonionic surfactant.

Advantages of the Invention

The present invention was completed by paying an attention to α-tocopherol acetate having a superior stability instead of unstable α-tocopherol, in order to solve the above described problems in the prior art. An advantage of the present invention is to give resistance to washing by adhering α-tocopherol acetate on a fiber material without undue loss in the feel and the moisture absorption and release properties possessed by the fiber itself. Another advantage is to provide a functionalized fiber material having a skin care effect enabling sustained discharge α-tocopherol acetate by the action of a fat and oil component such as sebum present on a skin surface of the human body.

Description of the Preferred Embodiments of the Invention

The fiber material used in the present invention includes natural fibers such as cotton, wool, silk, etc., regenerated fibers such as rayon, polynosic, cellulose acetate, etc., and synthetic fibers such as polyester, nylon, acrylic, etc., and may be a mixed fiber made of one or more kinds thereof. In addition, in the case of a regenerated fiber or a synthetic fiber, these fiber materials may contain substances to express other functional properties. The substances are not specially limited so long as they do not impede the sustained discharge function of α-tocopherol acetate. The form of the fiber material may be any of, for example, raw fiber, spun yarn, knitted or woven fabric, as well as a sewn fiber product. In the case where the fiber material is a raw fiber, spun yarn or knitted or woven fabric, it may be suitable for use as clothing.

α-tocopherol acetate used in the present invention may be an extract from natural products (d-α-tocopherol acetate) or a synthetic compound (d,l-α-tocopherol acetate), and is not specially limited to either. Further, particularly in the case of d-α-tocopherol acetate which is an extract from natural products, the α-tocopherol acetate may be mixed with a homologue such as β-tocopherol and γ-tocopherol or tocotrienol.

The surfactant to emulsify the above described α-tocopherol acetate in water is an anionic surfactant alone or a mixture of an anionic surfactant and a nonionic surfactant. The anionic surfactant used is not specially limited, and includes, for example, various kinds of fatty acid soaps, sodium lauryl sulfate, sodium higher alcohol sulfate, sodium dodecylbenzenesulfonate, sodium dialkylphosphosuccinate, calcium alkylphosphate, sodium polyoxyethylenealkylether sulfate, and the like. The nonionic surfactant, preferably to be used in combination with an anionic surfactant includes, for example, polyoxyethylene derivatives, sorbitan monoalkylate, sorbitan dialkylate, sorbitan trialkylate, and the like. The surfactant is not specially limited, but may be suitably selected from these surfactants. Further, the mixing ratio of an anionic surfactant and a nonionic surfactant is not specially limited, but is preferably nearly equal.

A method for manufacturing a functionalized fiber material of the present invention is as follows. Firstly, an emulsion containing α-tocopherol acetate is prepared by emulsifying α-tocopherol acetate in water with a surfactant by mixing and stirring. A fiber material is dipped in the emulsion, squeezed at a squeeze ratio of 60 to 120 %, then dried and treated at 80 to 200°C for 1 to 30 minutes.

The concentration of α-tocopherol acetate in the emulsion when a fiber material is treated is preferably in a range of 0.5 to 100.0 g/l. A concentration out of this range is not preferable because α-tocopherol acetate hardly remains after washing at a concentration lower than 0.5 g/l, and emulsification becomes difficult at a concentration over 100.0 g/l.

The concentration of surfactant in the emulsion is not specially limited so long as the concentration is within a range allowing emulsification. The preferred concentration is as low as possible within the range allowing emulsification, because too high a concentration of surfactant to the amount of α-tocopherol acetate used lowers the resistance to washing. The method of stirring is not specially limited and may be stirring using, for example, a homogenizer.

Further, in emulsifying, α-tocopherol acetate can be emulsified alone. More preferably however an organic solvent can be used in combination in order to adjust the concentration and viscosity of the emulsion. Any organic solvent may be used here so long as the solvent can dissolve α-tocopherol acetate. For example, oleic acid, squalane and derivatives thereof, hexane, dimethylether, ethyl acetate and dodecanol and the like are preferably used, alone or in mixture of two or more of them.

A functionalized fiber material obtained by the method of the present invention and having adhered α-tocopherol acetate can have a superior resistance to washing, for example, α-tocopherol acetate of not less than 50% can remain after 10 repeated washings by a conventional method. This is presumed to be caused by very low hydrophilicity of α-tocopherol acetate emulsion, as well as by impregnating α-tocopherol acetate into the inside of the fiber material.

According to the present invention, α-tocopherol acetate adhered to a functionalized fiber material is presumed to be gradually dissolved in a fat and oil component such as sebum remaining on the skin surface of the human body in wearing, thus being sustainedly discharged on to the skin surface, and to express a skin care effect by being incorporated into the skin. Furthermore, a functionalized fiber material manufactured according to the present invention can give little effect on feeling and moisture absorption and release properties essentially possessed by the fiber material.

Examples

Hereinunder, the present invention will be explained more concretely but the present invention is not limited to being within the range of these examples. Each of measured values was determined according to the test methods described below.

*Evaluation of feeling*

The feeling of woven fabrics which were woven from functionalized fiber materials of the present invention were judged by means of a tactile test by 10 examiners. Judgment was made according to the following criteria based on the total scores calculated from the evaluation results of each examiner which were made giving 1 point for a good feeling and 0 point for a bad feeling.

① 8 - 10 Points O (superior)

② 4 - 7 Points △ (good)

③ 0 - 3 Points × (bad)

*Coefficients of moisture absorption and discharge*

About 1 g of a sample was put into a weighing bottle having a weight of $W_h$ g, dried at 105°C for 60 minutes with its cap being open, cooled by leaving in a desiccator containing silica gel, then weighed as $W_0$ g. Subsequently, the sample in the bottle was kept in a desiccator which was kept at 60 % RH overnight, then kept in a thermo-hygrostat being conditioned at 35°C and 90% RH with the cap opened. After 60 minutes, the weighing bottle was taken out with the cap closed to be weighed as $W_2$ g. From these results, the coefficients of moisture absorption and release were calculated according to the following formulas(1) and (2), respectively.

$$\text{Coefficient of moisture absorption (\%)} = \frac{\{W_1\ (g)\ -\ W_0\ (g)\}}{\{W_0\ (g)\ -\ W_h\ (g)\}}\ \times\ 100 \quad (1)$$

$$\text{Coefficient of moisture release (\%)} = \frac{\{W_1\ (g)\ -\ W_2\ (g)\}}{\{W_0\ (g)\ -\ W_h\ (g)\}}\ \times\ 100 \quad (2)$$

*Method for measuring adhered amounts of d,l-α-tocopherol acetate and α-tocopherol*

A sample was dipped in an isopropanol solution, followed by shaking at 37°C for 2 hours. The supernatant was then analyzed by high performance liquid chromatography (HPLC). The amount of d,l-α-tocopherol acetate adhered to the sample was calculated from the area of the peak of d,l-α-tocopherol acetate obtained. By conducting the same procedures, an amount of α-tocopherol adhered to the sample was calculated from an area of the peak of α-tocopherol obtained.

*Evaluation of sustained discharge property*

A sample was added to oleic acid so that the addition amount became 0.3 to 0.5 % o.w.f., then washed with a commercially available detergent (trade name: Attack, made by Kao Co., Ltd.) according to the method of 2.2 (1) Washing method No. 103 in JIS L0217, "Care labelling of textile goods". After washing 1, 4 and 10 times, amounts of d,l-α-tocopherol acetate and α-tocopherol remaining on the sample after drying were determined according to the above described measuring method for adhered amounts.

Example 1

Seven kinds of treatment liquids were prepared by mixing d,l-α-tocopherol acetate (made by Kanto Chemical Co., Inc.), squalane, dodecanol. an anionic surfactant (trade name: Levenol WX, made by Kao Corp.) and a nonionic surfactant (trade name: Leodol, made by Kao Corp.) according to the recipes described in Table 1, respectively, adding water so that total quantities became 1 kg, respectively, then emulsifying them with a homogenizer. As for the comparative treatment liquid 7, a usable treatment liquid could not be obtained because the emulsification was difficult due to the concentration of d,l-α-tocopherol being as high as 150 g/l. For comparison, a further comparative treatment liquid was prepared, which was made by dissolving only 1 g of α-tocopherol so that the total quantity became 1 kg.

Table 1

| | Treatment liquid 1 Comparative | Treatment liquid 2 | Treatment liquid 3 | Treatment liquid 4 | Treatment liquid 5 | Treatment liquid 6 | Treatment liquid 7 Comparative |
|---|---|---|---|---|---|---|---|
| d,1-α-tocopherol | 0.3 | 0.5 | 1.0 | 1.0 | 50.0 | 100.0 | 150.0 |
| Squalane (g) | 1.5 | 2.5 | 5.0 | 5.0 | 0 | 0 | 0 |
| Dodecanol (g) | 0.3 | 0.5 | 1.0 | 1.0 | 50.0 | 100.0 | 150.0 |
| Anionic surfactant (g) | 0.2 | 0.4 | 0.8 | 1.5 | 40.0 | 80.0 | 100.0 |
| Nonionic surfactant (g) | 0.2 | 0.4 | 0.8 | 0 | 40.0 | 80.0 | 100.0 |
| water (g) | 997.5 | 995.7 | 991.4 | 991.5 | 820.0 | 640.0 | 460.0 |

Test fabrics having a size of 30 cm x 30 cm were cut out from a broad woven fabric made of cotton 100 % [(11.81 tex x 11.81 tex) / (144 yarns / inch x 76 yarns / inch)] treated by singeing, desizing, scouring and bleaching under the usual conditions. Each of the test fabrics was dipped in the treatment liquids 1 to 6, respectively, then squeezed at a squeeze ratio of 90 %, followed by a heat treatment at 120°C for 5 minutes to give samples 1 to 6. In addition, a further comparative sample was obtained by dipping the test fabric in the comparative treatment liquid followed by the same treatments as described above.

The samples 1 to 6 and the further comparative sample were washed 10 times using a domestic electric washing machine and a home detergent (trade name: Attack, made by Kao Corp.) according to JIS L0217, Washing No. 103. Addition amounts of d,l-α-tocopherol acetate and α-tocopherol were measured for the samples 1 to 6 and the further comparative sample in the initial stage (before washing) and after 10 washings. In addition, evaluations of the sustained discharge property were performed with the samples for evaluation of the sustained discharge property prepared by conducting 1, 4 and 10 washings then drying. Furthermore, evaluations of feeling and moisture absorption and release properties were conducted for an untreated broad woven fabric, the samples 1 to 6 and the further comparative sample. The results are shown in Table 2.

Table 2

| | Adhered amount (mg/g) | | Sustained discharge property (mg/g) | | | Moisture absorbing and releasing properties | | Feeling |
|---|---|---|---|---|---|---|---|---|
| | Init. stage | After 10W[1]) | After 1W[1]) | After 4W[1]) | After 10W[1]) | Coeff. of moist. abs. (%) [2]) | Coeff. of moist. rel. (%)[3]) | |
| Sample 1 comparative | 0.10 | 0 | 0 | 0 | 0 | 12.5 | 6.9 | O |
| Sample 2 | 0.30 | 0.13 | 0.15 | 0.05 | 0.02 | 12.5 | 6.9 | O |

Table continued

| | Adhered amount (mg/g) | | Sustained discharge property (mg/g) | | | Moisture absorbing and releasing properties | | Feeling |
|---|---|---|---|---|---|---|---|---|
| | Init. stage | After 10W[1] | After 1W[1] | After 4W[1] | After 10W[1] | Coeff. of moist. abs. (%) [2] | Coeff. of moist. rel. (%)[3] | |
| Sample 3 | 0.46 | 0.25 | 0.18 | 0.10 | 0.06 | 12.3 | 6.8 | O |
| Sample 4 | 0.43 | 0.22 | 0.17 | 0.11 | 0.07 | 12.3 | 6.8 | O |
| Sample 5 | 20.30 | 10.20 | 8.90 | 3.00 | 1.50 | 12.2 | 6.5 | O |
| Sample 6 | 45.00 | 21.30 | 15.60 | 5.80 | 2.30 | 12.2 | 6.6 | O |
| Comparative Sample | 0.55 | 0 | 0 | 0 | 0 | 12.6 | 6.9 | O |
| Untreated fabric | - | - | - | - | - | 12.7 | 6.9 | O |
| 1) 1W, 4W and 10W mean 1, 4 and 10 washings, respectively<br>2) Coeff. of moist. abs. (%) means Coefficient of moisture absorption (%)<br>3) Coeff. of moist. rel. (%) means Coefficient of moisture release (%) | | | | | | | | |

In Table 2, samples 2 to 6, which were treated with d,l-$\alpha$-tocopherol acetate emulsions containing 0.5 to 100.0 g/l of d,l-$\alpha$-tocopherol acetate, had 0.3 to 45.0 mg/g of adhered d,l-$\alpha$-tocopherol acetate based on the weights of samples. Not less than 50 % of d,l-$\alpha$-tocopherol acetate remained in any sample even after 10 washings proving that the samples have an excellent resistance to washing. Further, in the evaluation test for sustained release in which addition of oleic acid and washing were repeated, the sustained discharge property of the compound was confirmed.

On the other hand, comparative sample 1 treated with 0.3 g/l of d,l-$\alpha$-tocopherol acetate emulsion showed the adhered amount in the initial stage which was as low as 0.10 g/l, and no remaining d,l-$\alpha$-tocopherol acetate was found in the sample after 10 washings. In addition, although the furthercomparative sample treated with only 1 g/l of $\alpha$-tocopherol as a comparative treating liquid had an adhered amount of 0.55 mg/g of $\alpha$-tocopherol in the initial stage before washing, little $\alpha$-tocopherol was found in the adhered amount after 10 washings and on the samples after 1, 4 and 10 washings in the evaluation test for the sustained discharge property, proving no resistance to washing. All of these samples showed good feel as well as in the evaluation for the moisture absorption and release properties.

Example 2

3 kg of treatment liquid was prepared by the same procedures as for the treatment liquid 3 described in Table 1 in Example 1, which was divided into three treatment liquids of 1 kg each.

Test fabrics 30 cm $\times$ 30 cm were cut out from a broad woven fabric made of cotton 100 % [(11.81 tex $\times$ 11.81 tex) / (144 yarns / inch $\times$ 76 yarns / inch)] treated by singeing, desizing, scouring and bleaching under the usual conditions, from a broad woven fabric made of cotton 50 % mixed with polyester 50 % [(14.06 tex $\times$ 14.06 tex) / (110 yarns / inch $\times$ 57 yarns / inch)] treated by singeing, desizing, scouring and bleaching under the usual conditions, and from a filament woven fabric made of nylon 100 % [(7.8 tex $\times$ 7.8 tex) / (214 yarns / inch $\times$ 150 yarns / inch)] treated by singeing, desizing and scouring under the usual conditions, respectively. These three kinds of fabrics were dipped in the treatment liquid, then squeezed at a squeezing rate of 90 %, followed by heat treatment at 120°C for 5 minutes, to obtain samples 7, 8 and 9, corresponding to a broad woven fabric made of cotton 100 %, a broad woven fabric made of cotton mixed with polyester and a filament woven fabric made of nylon 100 %, respectively. The samples 7, 8 and 9 were washed 10 times using a domestic electric washing machine and a home detergent (trade name: Attack, made by Kao Corp.) according to JIS L0217, Washing No. 103. Adhered amounts of d,l-$\alpha$-tocopherol acetate were measured with the samples 7, 8 and 9 in their initial stages and after 10 washings. In addition, an evaluation test for sustained discharge property was conducted with the samples 7, 8 and 9 after 1, 4 and 10 washings and dryings, and also evaluations of feel and moisture absorption and release properties were conducted with the samples 7, 8, 9 and each untreated fabric. The results are shown in Table 3.

Table 3

| | Adhered amount (mg/g) | | Sustained discharge property (mg/g) | | | Moisture absorbing and releasing properties | | Feeling |
|---|---|---|---|---|---|---|---|---|
| | Init. stage | After 10M[1]) | After 1W[1]) | After 4W[1]) | After 10w[1]) | Coeff. of moist. abs.(%)[2]) | Coeff. of moist. rel. (%)[3]) | |
| Sample 7 | 0.46 | 0.25 | 0.18 | 0.10 | 0.06 | 12.3 | 6.8 | O |
| Untreated fabric | - | - | - | - | - | 12.7 | 6.9 | O |
| Sample 8 | 0.43 | 0.23 | 0.19 | 0.11 | 0.05 | 6.5 | 3.6 | O |
| Untreated fabric | - | - | - | - | - | 6.6 | 3.6 | O |
| Sample 9 | 0.50 | 0.30 | 0.22 | 0.13 | 0.07 | 4.5 | 2.5 | O |
| Untreated fabric | - | - | - | - | - | 4.6 | 2.5 | O |
| 1) 1W, 4W and 10W mean 1, 4 and 10 washings, respectively<br>2) Coeff. of moist. abs. (%) means Coefficient of moisture absorption (%)<br>3) Coeff. of moist. rel. (%) means Coefficient of moisture release (%) | | | | | | | | |

In Table 3, all of the samples 7, 8 and 9 which were treated with the d,l-$\alpha$-tocopherol acetate emulsion showed not less than 50% of remaining d,l-$\alpha$-tocopherol acetate even after 10 washings, clearly proving to have a very high resistance to washing. Moreover, the sustained discharge property was confirmed in the evaluation test on the sustained discharge property in which addition of oleic acid and washing were repeated. All of these samples showed good feel as well as little lowering in the evaluation of the moisture absorption and release properties by this treatment.

Effect of the Invention

The present invention provides a method for manufacturing a functionalized fiber material comprising treating a fiber material with a liquid in which $\alpha$-tocopherol acetate is emulsified in water with a surfactant which is an anionic surfactant alone or a mixture of an ionic surfactant and a nonionic surfactant. The functionalized fiber material has a superior feel in the clothing field, and exhibits skin-care effect, that is, an effect sustainedly to discharge a skin care component by the action of a fat and oil such as sebum present on a surface of the human body with resistance to washing, without losing moisture absorption and release properties which are essentially possessed by the fiber material.

**Claims**

1. A method for manufacturing a functionalized fiber material comprising treating a fiber material with a liquid in which $\alpha$-tocopherol acetate is emulsified in water with a surfactant, wherein the surfactant is an anionic surfactant alone or a mixture of an anionic surfactant and a nonionic surfactant, and the concentration of the $\alpha$-tocopherol acetate in the emulsified liquid is 0.5 to 100.0 g/l, to obtain the functionalized fiber material to which 0.3 to 45.0 mg/g of $\alpha$-tocopherol acetate is adhered for exhibiting a skin-care effect.

2. A method according to claim 1, wherein the $\alpha$-tocopherol acetate is emulsified in water with the surfactant and an organic solvent.

3. A method according to claim 2, wherein the organic solvent comprises one or more of oleic acid, squalane and derivatives thereof, hexane, dimethylether, ethyl acetate and dodecanol.

**Patentansprüche**

1. Verfahren zur Herstellung eines funktionalisierten Fasermaterials, das das Behandeln eines Fasermaterials mit einer Flüssigkeit umfasst, in der ein α-Tocopherolacetat in Wasser mit einem Tensid emulgiert ist, wobei das Tensid ein anionisches Tensid alleine oder ein Gemisch aus einem anionischen Tensid und einem nichtionischen Tensid ist, und die Konzentration des α-Tocopherolacetats in der emulgierten Flüssigkeit 0,5 bis 100,0 g/l beträgt, um das funktionalisierte Fasermaterial zu erhalten, an das 0,3 bis 45,0 mg/g α-Tocopherolacetat gebunden ist, um einen Hautpflegeeffekt zu erzielen.

**2.** Verfahren nach Anspruch 1, wobei das α-Tocopherolacetat in Wasser mit dem Tensid und einem organischen Lösungsmittel emulgiert wird.

**3.** Verfahren nach Anspruch 2, wobei das organische Lösungsmittel Ölsäure, Squalan und Derivate davon, Hexan, Dimethylether, Ethylacetat und/oder Dodecanol umfasst.

**Revendications**

**1.** Méthode pour la fabrication d'un matériau fibreux fonctionnalisé comprenant le traitement d'un matériau fibreux avec un liquide où de l'acétate d'-α-tocophérol est émulsionné dans l'eau avec un agent tensio-actif, où l'agent tensio-actif est un agent tensio-actif anionique seul ou un mélange d'un agent tensio-actif anionique et d'un agent tensio-actif non ionique et la concentration de l'acétate d'-α-tocophérol dans le liquide émulsionné est de 0,5 à 100,0 g/l pour obtenir le matériau fibreux fonctionnalisé auquel adhère 0,3 à 45,0 mg/g d'acétate d'-α-tocophérol pour présenter un effet de soins pour la peau.

**2.** Méthode selon la revendication 1, où l'acétate d'-α-tocophérol est émulsionné dans l' eau avec l'agent tensio-actif et un solvant organique.

**3.** Méthode selon la revendication 2, où le solvant organique comprend un ou plusieurs d'acide oléique, squalane et dérivés, hexane, diméthyléther, acétate d'éthyle et dodécanol.